# EUROPEAN PATENT APPLICATION

(11) **EP 4 818 185 A1**
(43) Date of publication of application: **07.10.2026**
(21) Application number: 24885051.3
(22) Date of filing: 20.12.2024
(51) Int. Cl.: C07J 9/00, A61K 31/575, A61K 36/258, A61P 35/00, A61P 25/00, A61P 9/00

(54) **CRYSTAL FORMS OF 20(S)-PROTOPANAXADIOL, PREPARATION METHOD THEREFOR AND USE THEREOF**

(30) Priority: 30.10.2023 CN 202311424711
(71) Applicant: Shanghai Baocheng Tailai Technology Co., Ltd., Shanghai 200131 (CN)
(72) Inventor: WANG, Jihua, Shanghai 201203 (CN); HAN, Wei, Shanghai 201203 (CN); JIA, Weiguo, Shanghai 201203 (CN); FU, Wenbao, Shanghai 201203 (CN); YANG, Zirong, Shanghai 201203 (CN)
(74) Representative: The IP Asset Partnership Limited
(86) International application number: PCT/CN2024/141102
(87) International publication number: WO 2025/093056

(57) **Abstract**

The present application relates to the field of medicine, and specifically relates to a crystal form A and crystal form B of 20(S)-protopanaxadiol, a preparation method therefor and the use thereof. The crystal form A and crystal form B provided in the present application are different from the existing known crystal forms of 20(S)-protopanaxadiol, each have a distinct profile, can be perfectly reproduced, have good bulk density, fluidity and stability, and good characteristics in terms of fighting depression, anxiety, and cognitive disorder, preventing and/or treating Alzheimer's disease (AD), amyotrophic lateral sclerosis (ALS), and treating diseases such as tumors or cardiovascular diseases, and have broad application prospects in the drug development of 20(S)-protopanaxadiol.

## Description

### Cross Reference

The present application claims priority to Chinese Patent Application No. 2023114247117, filed on October 30, 2023, entitled "CRYSTAL FORMS OF 20(S)-PROTOPANAXADIOL, PREPARATION METHOD THEREFOR AND USE THEREOF", the entire disclosure of which is incorporated herein by reference.

### Technical Field

The present application relates to the field of medicine, specifically to crystal forms of 20(S)-protopanaxadiol, a preparation method therefor and the use thereof.

### Background Art

20(S)-protopanaxadiol, also abbreviated as 20(S)-PPD, is a plant component with various pharmacological active functions such as inhibiting cancer cell growth, anti-depression, anti-anxiety, anti-cognitive disorder, preventing and/or treating Alzheimer's disease (AD), amyotrophic lateral sclerosis (ALS), and cardiovascular diseases. Its molecular formula is C₃₀H₅₂O₃, with a structural formula as follows:

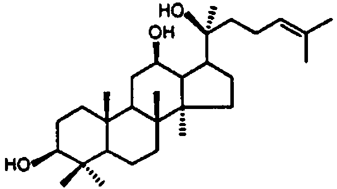

Currently, there is little research on the crystal forms of 20(S)-protopanaxadiol. Patent Publication No. CN115991729A discloses two crystal forms of 20(S)-protopanaxadiol, which were prepared by heating and dissolving with acetone and acetonitrile, and then cooling to crystallize, respectively.

In addition, existing studies have also disclosed some preparation methods of 20(S)-protopanaxadiol. Most of these preparation methods use plant (such as Panax ginseng, Panax notoginseng, red ginseng, American ginseng, and Gynostemma pentaphyllum) saponins as raw materials, to obtain 20(S)-protopanaxadiol through techniques such as hydrolysis, followed by using alcohol as a main elution solvent system, and performing separation and purification with column chromatography to finally obtain 20(S)-protopanaxadiol in white powder form. However, the 20(S)-protopanaxadiol products (including the amorphous crystalline form of 20(S)-protopanaxadiol) obtained by the above methods still have disadvantages such as low bulk density, low solubility, and poor mechanical stability.

In view of this, the present application is proposed.

### Summary

The present application provides new crystal forms of 20(S)-protopanaxadiol, preparation methods therefor and the use thereof, specifically as follows:
In a first aspect, the present application provides a crystal form A of 20(S)-protopanaxadiol.

In the present application, the X-ray powder diffraction pattern of the crystal form A, represented by 2θ ± 0.2° diffraction angles, exhibits characteristic diffraction peaks at least at 6.5°, 4.2°, and 15.7°.

Preferably, the X-ray powder diffraction pattern of the crystal form A, represented by 2θ ± 0.2° diffraction angles, further exhibits characteristic diffraction peaks at
(1) at least one of 17.1°, 18.6°, and 13.1°; and/or
(2) at least one of 17.9°, 10.6°, and 20.7°.

In a preferred embodiment, the X-ray powder diffraction pattern of the crystal form A, represented by 2θ ± 0.2° diffraction angles, exhibits characteristic diffraction peaks at least at 6.5°, 4.2°, 15.7°, 17.1°, 18.6°, 13.1°, 17.9°, 10.6°, and 20.7°.

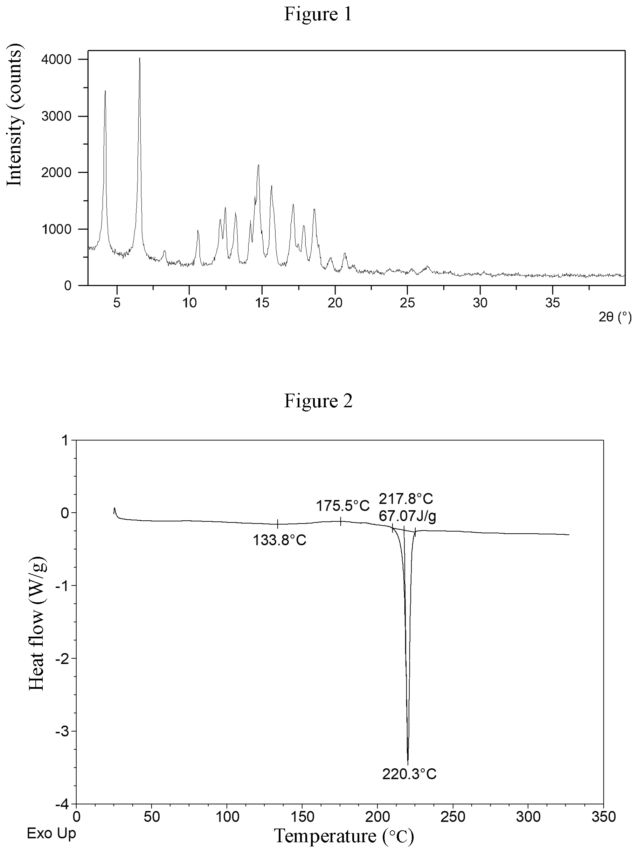

In a further preferred embodiment, the crystal form A of 20(S)-protopanaxadiol according to the present application has an X-ray powder diffraction pattern basically as shown in Figure 1.

The crystal form A is characterized by peak positions in pattern (Bragg angle 2θ, expressed in degrees), relative intensity (%), and interplanar spacing d, with the data substantially shown in table 1:

**Table 1**

| Diffraction angle 2θ | d Value | Relative intensity % |
|---|---|---|
| 4.2 | 21.2 | 82.3 |
| 6.5 | 13.5 | 100.0 |
| 10.6 | 8.4 | 18.0 |
| 13.1 | 6.7 | 27.9 |
| 15.7 | 5.6 | 41.1 |
| 17.1 | 5.2 | 31.2 |
| 17.9 | 5.0 | 21.3 |
| 18.6 | 4.8 | 30.8 |
| 20.7 | 4.3 | 9.1 |

In the present application, the melting point of the crystal form A is 219 to 221°C.

When performing differential scanning calorimetry, the crystal form A shows an endothermic signal when heated to around 133°C and an exothermic signal when heated to around 175°C, and begins to melt when heated to around 220°C. Its DSC thermogram is as shown in figure 2.

The crystal form A of 20(S)-protopanaxadiol provided by the present application has a distinct profile that can be perfectly reproduced. It has the characteristics of high bulk density and strong fluidity, especially exhibits superior characteristics in terms of mechanical stability, solid-state stability, and the like. It shows higher solubility in FaSSIF (fasted state simulated intestinal fluid) and has broad application prospects in the drug development of 20(S)-protopanaxadiol.

In a second aspect, the present application provides a preparation method for the crystal form A.

In the present application, the preparation method for the crystal form A comprises: dissolving crude 20(S)-protopanaxadiol in a good solvent to obtain a 20(S)-protopanaxadiol solution, adding an antisolvent dropwise to the 20(S)-protopanaxadiol solution until a gel-like substance is formed, and subjecting the gel-like substance to at least one temperature cycle;
wherein, the crude 20(S)-protopanaxadiol of the present application is a known compound that can be obtained through various channels.

In an optional embodiment of the present application, the crude 20(S)-protopanaxadiol can be selected from the "protopanaxadiol" prepared by the preparation method described in the invention patent with patent publication number CN1252082C (preparation methods for protopanaxadiol and protopanaxatriol); or selected from the "crystal form A" described in the invention patent with patent publication number CN115991729A (20(S)-protopanaxadiol crystal form A, crystal form B, and preparation methods thereof). After detection and characterization, the crystal form of the "protopanaxadiol" and the "crystal form A" in CN115991729A mentioned above is actually the "crystal form D" as described in the present application (see "Comparative Examples and Experimental Examples" described later herein).

In a more specific embodiment provided by the present application, the crude 20(S)-protopanaxadiol is preferably prepared by the method provided in Example 3 of the invention patent with publication number CN1252082C (preparation methods of protopanaxadiol and protopanaxatriol).

In the preparation method for the crystal form A described in the present application, crude 20(S)-protopanaxadiol is first dissolved in a good solvent to obtain a 20(S)-protopanaxadiol solution;
wherein, the good solvent can be selected from known solvents that can dissolve 20(S)-protopanaxadiol well, preferably at least one selected from methyl isobutyl ketone, anisole, or chloroform, more preferably methyl isobutyl ketone.

In order to further control the dissolved state of the solute, the ratio of the amount of the crude 20(S)-protopanaxadiol to the amount of the good solvent is preferably 10-30 mg : 0.5-1.0 mL, more preferably 18-22 mg : 0.60.8 mL.

In the preparation method provided by the present application, after a 20(S)-protopanaxadiol solution is obtained, preferably a filter membrane with a pore size of 0.4 to 0.5 µm is further used to filter the 20(S)-protopanaxadiol solution.

In the present application, the above filtration operation can not only achieve conventional impurity removal, but also effectively eliminate the influence of impurities on the formation of microstructure of crystals during the subsequent gas-liquid permeation crystallization process, thereby promoting the smoother precipitation of the crystal form A from the solution of the crude 20(S)-protopanaxadiol.

The pore size of the filter membrane described in the present application is preferably 0.44 to 0.46 µm, more preferably 0.45 µm.

In the present application, the material of the filter membrane is not particularly limited, so long as it is from conventional commercial sources in the art.

In a more specific embodiment provided by the present application, the filter membrane is selected from polytetrafluoroethylene filter membranes.

In the preparation method provided by the present application, preferably, the obtained 20(S)-protopanaxadiol solution is stirred to obtain a clear solution. Preferably, the stirring speed is 500 to 2000 r/min, more preferably 1000 r/min.

Furthermore, in the present application, an antisolvent is added dropwise to the 20(S)-protopanaxadiol solution to form a gel-like substance; and then, the gel-like substance is subjected to at least one temperature cycle;
wherein, the antisolvent selection may be any solvent that is unfavorable for 20(S)-protopanaxadiol, particularly preferably at least one selected from n-heptane, n-hexane or n-pentane, more preferably n-heptane.

In the present application, the ratio of the amount of the antisolvent to the amount of the good solvent is preferably 5 mL : 0.5-1.0 mL, more preferably 5 mL : 0.6-0.8 mL, to better promote the formation of crystals.

In the preparation method provided by the present application, temperature control is one of the key factors for the formation of high-quality crystals. The dissolved state of 20(S)-protopanaxadiol in the good solvent and antisolvent described in the present application will be affected by temperature to some extent, and appropriate temperature control is very favorable for the crystal maturation and crystallization of 20(S)-protopanaxadiol.

Preferably, the temperature cycle comprises: heating the gel-like substance to 45-55°C, holding at a constant temperature for 1 to 3 hours, cooling at a rate of 0.05 to 1°C/min to 4 to 6°C, and holding at a constant temperature for crystal maturation for 1 to 3 hours, wherein, preferably, the heating is uniformly heating to 45°C to 55°C within 10 to 40 min; and preferably, the cooling rate is 0.1°C/min to 0.5°C/min.

In a preferred embodiment provided by the present application, the temperature cycle comprises: heating the gel-like substance to 50°C within 0.3 to 0.5 hours, holding at a constant temperature for 2 hours, then cooling at a rate of 0.1 to 0.5 °C/min to 5°C, and holding at a constant temperature for crystal maturation for 2 hours.

In the preparation method provided by the present application, while the gel-like substance is subjected to a temperature cycle, preferably, the gel-like substance is stirred except the stage of holding at a constant temperature for crystal maturation (i.e., the heating, heating to 45°C to 55°C, holding at a constant temperature and cooling steps are all carried out under stirring); preferably, the stirring speed is 800 to 1200 r/min; more preferably 1000 r/min.

The specific operation mode of the stirring described in each step of the present application is not particularly limited, and conventional stirring operations in the art, such as magnetic stirring, can be used.

In the present application, the number of the temperature cycles is preferably 2 to 4 times. After the temperature cycle is completed, a precipitated solid crystal form A can be obtained.

In the present application, preferably the precipitated solid crystal form A is subjected to filtering and drying, and the filtering and drying steps can be carried out using conventional methods in the art, not being particularly limited in the present application.

As the best embodiment of the present application, preferably the preparation method comprises the following steps:
weighing approximately 20 mass units of solids of crude 20(S)-protopanaxadiol and placing in a glass bottle, adding 0.6 volume units of methyl isobutyl ketone to dissolve the 20(S)-protopanaxadiol crude solid, filtering the sample solution into a new glass bottle using a polytetrafluoroethylene filter membrane with a pore size of 0.45 µm, followed by magnetic stirring (at a speed of approximately 1000 revolutions per minute) to obtain a clear solution, adding 5 volume units of n-heptane dropwise to obtain a gel-like sample, and transferring the sample to temperature cycle conditions for crystal maturation and crystallization.

In the preparation method described in the present application, unless otherwise specified, when the mass unit is mg, the corresponding volume unit is mL.

The specific temperature cycle conditions are as follows:
first cycle: uniformly heating to 50°C in 0.3 hours, holding at a constant temperature of 50°C for 2 hours; cooling at a rate of 0.5°C/min to 5°C and holding at a constant temperature of 5°C for 2 hours;
second cycle: uniformly heating to 50°C in 0.4 hours, holding at a constant temperature of 50°C for 2 hours; cooling at a rate of 0.3°C/min to 5°C and holding at a constant temperature of 5°C for 2 hours; and
third cycle: uniformly heating to 50°C in 0.5 hours, holding at a constant temperature of 50°C for 2 hours; cooling at a rate of 0.1°C/min to 5°C and holding at a constant temperature of 5°C for 2 hours.

In the above three cycles, except for holding at a constant temperature of 5°C for 2 hours for static crystal maturation, all other steps are carried out under magnetic stirring.

After a total of 3 cycles, solids were precipitated, and the resulting solids were subjected to centrifugal separation to obtain a crystal form A.

The above preparation method provided by the present application can perfectly reproduce a crystal form A of 20(S)-protopanaxadiol, and the product quality is stable and reliable, which plays an important role in the subsequent development and application of the crystal form A of 20(S)-protopanaxadiol.

The X-ray powder diffraction pattern of the crude 20(S)-protopanaxadiol prepared in Example 3 of the invention patent with publication number CN1252082C (preparation methods for protopanaxadiol and protopanaxatriol) is shown in figure 3.

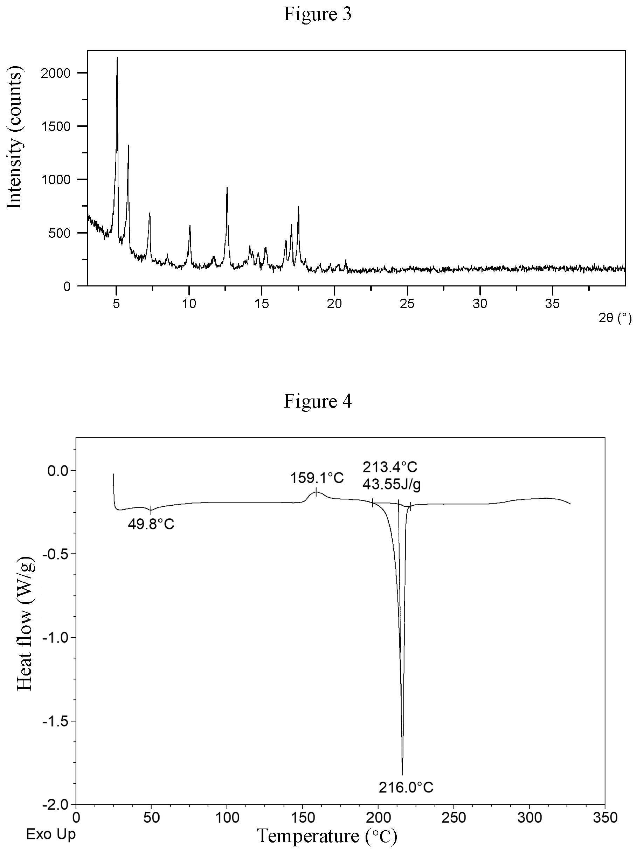

As shown in figure 3, the X-ray powder diffraction pattern of the crude 20(S)-protopanaxadiol, represented by 2θ ± 0.2° diffraction angles, exhibits characteristic diffraction peaks at 5.0°, 5.8°, 12.6°, 17.5°, 7.3°, 17.0°, 10.0°, 16.6°, and 15.3°.

The crude 20(S)-protopanaxadiol represented by figure 3 is characterized by the peak positions in pattern (Bragg angle 2θ, expressed in degrees), relative intensity (%), and interplanar spacing d, with the data substantially shown in table 2:

**Table 2**

| Diffraction angle 2θ | d Value | Relative intensity % |
|---|---|---|
| 5.0 | 17.6 | 100.0 |
| 5.8 | 15.2 | 60.8 |
| 7.3 | 12.2 | 26.8 |
| 10.0 | 8.8 | 22.2 |
| 12.6 | 7.0 | 43.7 |
| 15.3 | 5.8 | 12.6 |
| 16.6 | 5.3 | 17.6 |
| 17.0 | 5.2 | 23.9 |
| 17.5 | 5.1 | 32.7 |

The crude 20(S)-protopanaxadiol has a melting point of 215 to 217°C.

When performing differential scanning calorimetry, the crude 20(S)-protopanaxadiol shows an endothermic signal when heated to around 50°C and an exothermic signal when heated to around 159°C, and begins to melt when heated to around 216°C. Its DSC thermogram is as shown in figure 4.

The crude 20(S)-protopanaxadiol can also be represented by a crystal form D in the present application. Comparing the information such as X-ray powder diffraction pattern (i.e. figure 3) and DSC graph (i.e. figure 4) of the crude 20(S)-protopanaxadiol (crystal form D) with the information such as X-ray powder diffraction pattern (i.e. figure 1) and DSC graph (i.e. figure 2) of the crystal form A of the 20(S)-protopanaxadiol claimed in the present application, it can be concluded that the crystal form A obtained in the present application is different from the existing known crystal form of 20(S)-protopanaxadiol.

In a third aspect, the present application provides another crystal form B of 20(S)-protopanaxadiol.

In the present application, the X-ray powder diffraction pattern of the crystal form B, represented by 2θ ± 0.2° diffraction angles, exhibits characteristic diffraction peaks at least at 7.5°, 14.4°, and 15.4°.

Preferably, the X-ray powder diffraction pattern of the crystal form B, represented by 2θ ± 0.2° diffraction angles, further exhibits characteristic diffraction peaks at
(1) at least one of 4.6°, 3.8°, and 16.9°; and/or
(2) at least one of 18.1°, 6.7°, and 13.1°.

In a preferred embodiment, the X-ray powder diffraction pattern of the crystal form B, represented by 2θ ± 0.2° diffraction angles, exhibits characteristic diffraction peaks at least at 7.5°, 14.4°, 15.4°, 4.6°, 3.8°, 16.9°, 18.1°, 6.7°, and 13.1°.

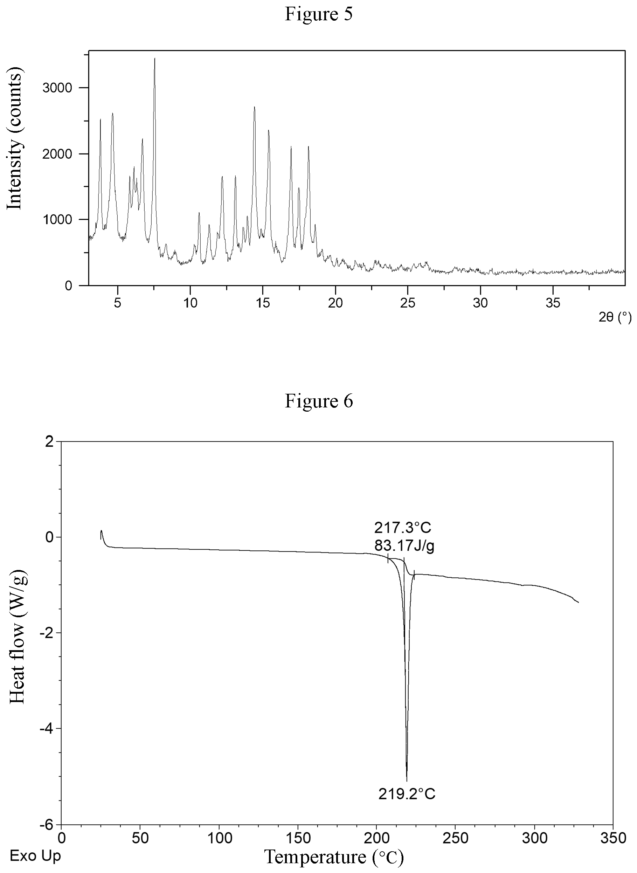

In a further preferred embodiment, the crystal form B of 20(S)-protopanaxadiol according to the present application has an X-ray powder diffraction pattern basically as shown in figure 5.

The crystal form B is characterized by the peak positions in pattern (Bragg angle 2θ, expressed in degrees), relative intensity (%), and interplanar spacing d, with the data substantially as shown in table 3:

**Table 3**

| Diffraction angle 2θ | d Value | Relative intensity % |
|---|---|---|
| 3.8 | 23.2 | 63.5 |
| 4.6 | 19.0 | 68.1 |
| 6.7 | 13.2 | 56.0 |
| 7.5 | 11.7 | 100.0 |
| 13.1 | 6.8 | 46.0 |
| 14.4 | 6.1 | 78.9 |
| 15.4 | 5.8 | 69.0 |
| 16.9 | 5.2 | 60.9 |
| 18.1 | 4.9 | 60.0 |

In the present application, the melting point of the crystal form B is 218 to 220°C.

When performing differential scanning calorimetry, the crystal form B begins to melt when heated to around 219°C. Its DSC thermogram is as shown in figure 6.

The crystal form B of 20(S)-protopanaxadiol provided by the present application has a distinct profile that can be perfectly reproduced. It has the characteristics of high bulk density, strong fluidity, and good solid-state stability.

In a fourth aspect, the present application provides a preparation method for the crystal form B.

In the present application, the preparation method for the crystal form B comprises: first heating crude 20(S)-protopanaxadiol to 160°C to 180°C, holding for 2 to 5 minutes; then cooling to 25°C to 35°C to obtain the crystal form B;
wherein, the crude 20(S)-protopanaxadiol is also an existing known compound (crystal form D). In a more specific embodiment provided by the present application, the crude 20(S)-protopanaxadiol is prepared by the method provided in Example 3 of the invention patent with the publication number CN1252082C (preparation methods for protopanaxadiol and protopanaxatriol).

In the preparation method for the crystal form B according to the present application, the heating is preferably carried out in an inert gas environment, wherein, the inert gas is preferably N₂.

In the present application, the heating rate is preferably 8-15°C/min, more preferably 10-12°C/min. The cooling rate is preferably 25-35°C/min, more preferably 30°C/min.

As the best embodiment of the present application, preferably the preparation method comprises the following steps:
placing solids of crude 20(S)-protopanaxadiol in a DSC aluminum dish, heating at a heating rate of 10-15°C/min to 170°C under nitrogen purging conditions, holding at a constant temperature for 3 minutes, then cooling at a cooling rate of 30°C/min to 30°C, and collecting solids.

Comparing the information such as X-ray powder diffraction pattern (i.e. figure 3) and DSC graph (i.e. figure 4) of the crude 20(S)-protopanaxadiol with the information such as X-ray powder diffraction pattern (i.e. figure 5) and DSC graph (i.e. figure 6) of the crystal form B of 20(S)-protopanaxadiol claimed in the present application, it can be concluded that the crystal form B obtained in the present application is different from the existing known crystal form of 20(S)-protopanaxadiol.

In a fifth aspect, the present application also provides a pharmaceutical composition comprising the above-mentioned crystal form A or crystal form B of 20(S)-protopanaxadiol, specifically the pharmaceutical composition including but not limited to powder for injection, tablets, capsules, microspheres, self-microemulsifying compositions, dry suspensions, freeze-dried powders, or infusions and the like.

The composition of the present application may further contain pharmaceutically acceptable excipient; more preferably, the pharmaceutically acceptable excipient includes at least one of a carrier, diluent, and excipient. For example, when it is a freeze-dried powder, the pharmaceutically acceptable carrier can be an excipient. When the composition is a tablet, the pharmaceutically acceptable carrier may be a disintegrant, excipient and the like.

The crystal form A or crystal form B of 20(S)-protopanaxadiol described in the present application has the same chemical formula structure (only the crystal form is different) as the crude 20(S)-protopanaxadiol, and it also exhibits ideal therapeutic effects in the treatment of depression, anxiety, cognitive disorder, prevention and/or treatment of Alzheimer's disease (AD), amyotrophic lateral sclerosis (ALS), tumor or cardiovascular diseases, etc.

Due to the ideal physical and chemical properties of the crystal form A or crystal form B of 20(S)-protopanaxadiol obtained by the present application, a person skilled in the art can foresee that after preparing it into various drug formulations, it can further provide stability and efficacy of the drug formulations, and the determination of specific dosage forms and prescriptions is mastered by a person skilled in the art.

Moreover, the present application further claims any one of the following uses of the aforementioned crystal form A or crystal form B of 20(S)-protopanaxadiol:
(1) for the manufacture of a medicament for the treatment of depression, anxiety, cognitive disorder, tumor, or cardiovascular diseases;
(2) for treating depression, anxiety, cognitive disorder, tumor, or cardiovascular diseases; and
(3) for the manufacture of a medicament for the prevention and/or treatment of Alzheimer's disease (AD) and amyotrophic lateral sclerosis (ALS).

The crystal form A or crystal form B of 20(S)-protopanaxadiol described in the present application exhibits excellent pharmaceutical activity in the treatment of depressant, anxiety, cognitive disorder, tumor, and cardiovascular diseases, prevention and/or the treatment of Alzheimer's disease (AD), amyotrophic lateral sclerosis (ALS), and the like. Moreover, the crystal form A or crystal form B of 20(S)-protopanaxadiol described in the present application exhibits superior crystal form characteristics such as bulk density, fluidity, and stability compared to the crude 20(S)-protopanaxadiol, offering advantages in the drug development of 20(S)-protopanaxadiol.

Beneficial effects:
The present application provides novel crystal form A and crystal form B of 20(S)-protopanaxadiol, and corresponding preparation method and use thereof. The novel crystal forms provided by the present application have distinct profiles, can be perfectly reproduced, exhibit good characteristics in bulk density, fluidity and stability, and have broad application prospects in the drug development of 20(S)-protopanaxadiol. The preparation methods provided by the present application are simple and easy to implement, facilitating widespread application.

### Brief Description of the Drawings

Figure 1 shows the X-ray powder diffraction pattern of the crystal form A of 20(S)-protopanaxadiol of the present application.
Figure 2 shows the differential scanning calorimetry thermogram of the crystal form A of 20(S)-protopanaxadiol of the present application.
Figure 3 shows the X-ray powder diffraction pattern of the crude 20(S)-protopanaxadiol (crystal form D) used in the present application.
Figure 4 shows the differential scanning calorimetry thermogram of the crude 20(S)-protopanaxadiol (crystal form D) used in the present application.
Figure 5 shows the X-ray powder diffraction pattern of the crystal form B of 20(S)-protopanaxadiol of the present application.
Figure 6 shows the differential scanning calorimetry thermogram of the crystal form B of 20(S)-protopanaxadiol of the present application.

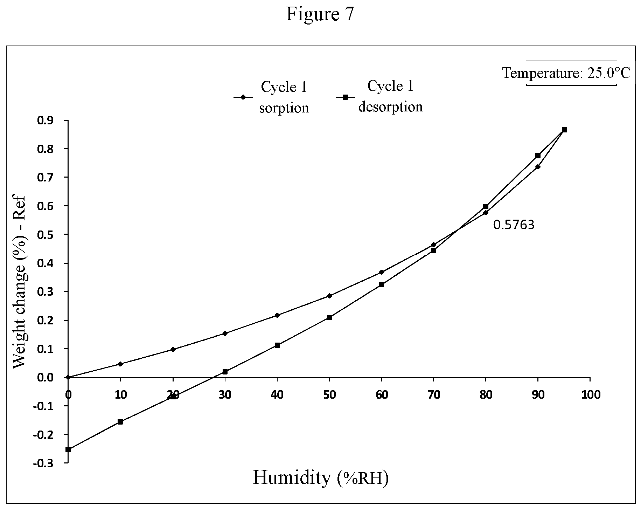

Figure 7 shows the dynamic vapor sorption profile of the crystal form A of 20(S)-protopanaxadiol of the present application.

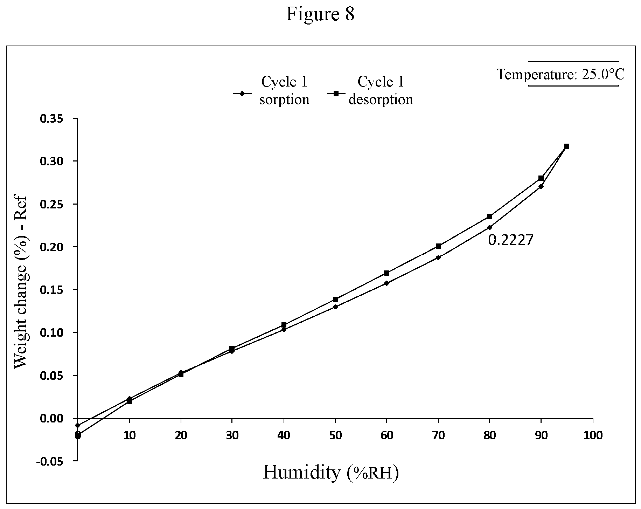

Figure 8 shows the dynamic vapor sorption profile of the crystal form B of 20(S)-protopanaxadiol of the present application.

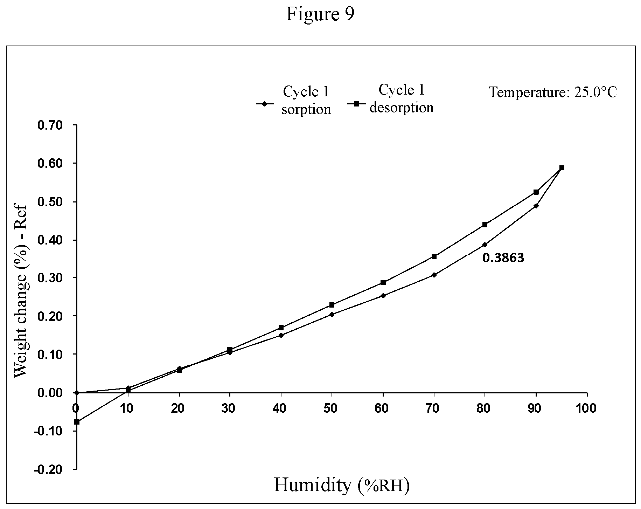

Figure 9 shows the dynamic vapor sorption profile of the crude 20(S)-protopanaxadiol (crystal form D) used in the present application.

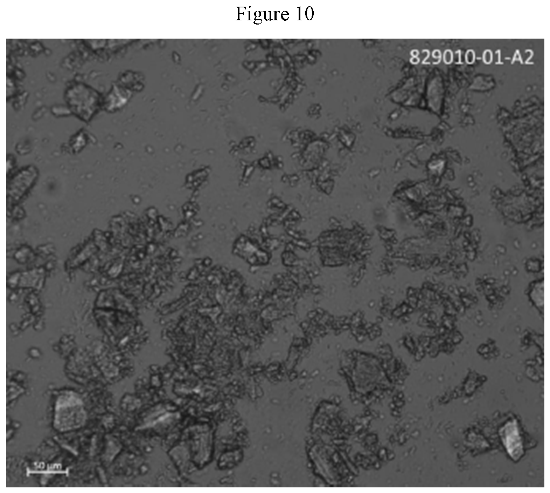

Figure 10 shows the PLM image of the crystal form A of 20(S)-protopanaxadiol of the present application.

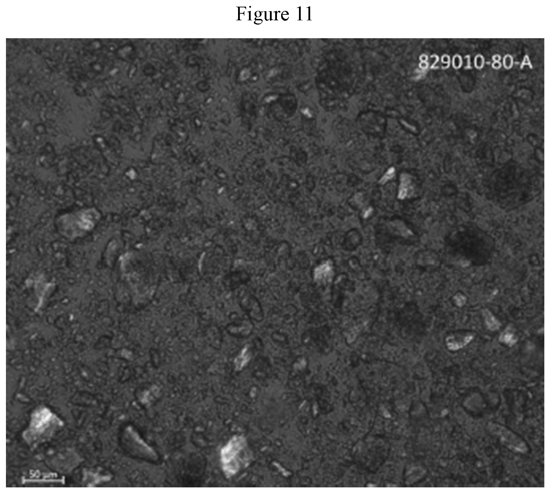

Figure 11 shows the PLM image of the crystal form B of 20(S)-protopanaxadiol of the present application.

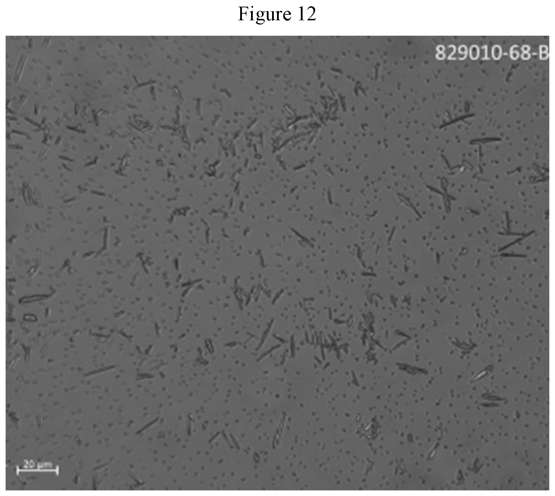

Figure 12 shows the PLM image of the crude 20(S)-protopanaxadiol (crystal form D) used in the present application.

### Specific Modes for Carrying Out the Embodiments

The present application will be further elaborated below in conjunction with specific Examples. It should be understood that these Examples are solely for illustrative purposes and not intended to limit the scope of the present application. Clearly, the described Examples represent only a portion of the Examples of the present application and not all Examples. Based on the Examples in the present application, all other Examples obtained by a person skilled in the art without creative effort fall within the scope of protection of the present application.

The abbreviations used in the present application are explained as follows:
XRPD: X-ray powder diffraction
DSC: Differential scanning calorimetry
TGA: Thermogravimetric analysis
DVS: Dynamic vapor sorption

The X-ray powder diffraction patterns described in the present application were collected on a PANalytical Empyrean or X'Pert3 X-ray powder diffraction instrument. The parameters of the X-ray powder diffraction method described in the present application are as follows:
X-ray source: Cu, Kα
Kα1 (Å):1.54060;Kα2 (Å):1.54439
Kα2/Kα1 intensity ratio: 0.50
Voltage: 45 kilovolts (kV)
Current: 40 milliamperes (mA)
Scanning range: from 3.0 to 40.0 degrees

The differential scanning calorimetry (DSC) thermogram described in the present application was collected on a TA Discovery 2500. The parameters of the differential scanning calorimetry (DSC) method described in the present application are as follows:
Scanning rate: 10°C/min
Protective gas: N₂

The thermogravimetric analysis (TGA) diagram described in the present application was collected on a TA Discovery 5500. The parameters of the thermogravimetric analysis (TGA) method described in the present application are as follows:
Scanning rate: 10°C/min
Protective gas: N₂

In the present application, the high-performance liquid chromatography (HPLC) purity data were obtained from a Thermo Fisher Vanquish Core, and the detector used was a diode array detector (DAD). The parameters of the HPLC method for testing purity described in the present application are as follows:
1. Chromatography column: Xselect CSH C18, 150 × 4.6 mm, 3.5 µm
2. Mobile phase:
A: Water
B: Acetonitrile
The elution gradient is as follows:

| Time (min) | B(%) |
|---|---|
| 0.0 | 80 |
| 16.0 | 80 |

3. Flow rate: 1.0 mL/min
4. Injection volume: 5 µL
5. Detection wavelength: 203 nm
6. Column temperature: 40°C
7. Injector temperature: room temperature
8. Diluent: acetonitrile

Unless otherwise specified, the following Examples are operated at room temperature.

### Example 1

Preparation of crude 20(S)-protopanaxadiol:
Crude 20(S)-protopanaxadiol was prepared using the method described in Example 3 of invention patent 200410018038.8 (preparation methods of protopanaxadiol and protopanaxatriol).

The X-ray powder diffraction pattern of the crude 20(S)-protopanaxadiol using Cu-Kα radiation is shown in figure 3.

The crude 20(S)-protopanaxadiol represented by figure 3 is characterized by the peak positions in pattern (Bragg angle 2θ, expressed in degrees), relative intensity (%), and interplanar spacing d, with the data shown in Table 2.

When performing differential scanning calorimetry, the crude 20(S)-protopanaxadiol showed an endothermic signal when heated to around 50°C and an exothermic signal when heated to around 159°C, and began to melt when heated to around 216°C. Its DSC thermogram is shown in figure 4.

### Example 2

Preparation of a crystal form A of 20(S)-protopanaxadiol:
The crude 20(S)-protopanaxadiol prepared in Example 1 was used as the raw material, approximately 20 mg of solids of the crude 20(S)-protopanaxadiol were weighed and placed in a 20 mL glass vial, and 0.6 mL of methyl isobutyl ketone was added to dissolve the solids of the crude 20(S)-protopanaxadiol. The sample solution was filtered into a new 20 mL glass vial using a polytetrafluoroethylene filter membrane with a pore size of 0.45 µm, followed by magnetic stirring (at a speed of approximately 1000 rpm) to obtain a clear solution, 5 ml of n-heptane was added dropwise therein to obtain a gel-like sample, and the sample was transferred to temperature cycle conditions for crystal maturation (except for the stage of holding at a constant temperature for crystal maturation, all other stages were carried out under magnetic stirring at a speed of approximately 1000 rpm).

The temperature cycle conditions were as follows:
first cycle: uniformly heating to 50°C in 0.3 hours, holding at a constant temperature of 50°C for 2 hours; cooling at a rate of 0.5°C/min to 5°C and holding at a constant temperature of 5°C for 2 hours;
second cycle: uniformly heating to 50°C in 0.4 hours, holding at a constant temperature of 50°C for 2 hours; cooling at a rate of 0.3°C/min to 5°C and holding at a constant temperature of 5°C for 2 hours; and
third cycle: uniformly heating to 50°C in 0.5 hours, holding at a constant temperature of 50°C for 2 hours; cooling at a rate of 0.1°C/min to 5°C and holding at a constant temperature of 5°C for 2 hours.

After a total of 3 cycles, solids were precipitated, and the resulting solids were subjected to centrifugal separation to obtain a crystal form A.

The X-ray powder diffraction pattern of the crystal form A of 20(S)-protopanaxadiol using Cu-Kα radiation is shown in figure 1.

The crystal form A of 20(S)-protopanaxadiol is characterized by the peak positions in pattern (Bragg angle 2θ, expressed in degrees), relative intensity (%), and interplanar spacing d, with the data shown in Table 1.

When performing differential scanning calorimetry, the crystal form A showed an endothermic signal when heated to around 133°C and an exothermic signal when heated to around 175°C, and began to melt when heated to around 220°C. Its DSC thermogram is as shown in figure 2.

### Example 3

Preparation of a crystal form A of 20(S)-protopanaxadiol:
The crude 20(S)-protopanaxadiol prepared in Example 1 was used as the raw material, approximately 19.8 mg of solids of the crude 20(S)-protopanaxadiol were weighed and placed in a 20 mL glass vial, and 0.8 mL of anisole was added to dissolve the solids of the crude 20(S)-protopanaxadiol. The sample solution was filtered into a new 20 mL glass vial using a polytetrafluoroethylene filter membrane with a pore size of 0.45 µm, followed by magnetic stirring (at a speed of approximately 1000 rpm) to obtain a clear solution, 5 ml of n-pentane was added dropwise therein to obtain a gel-like sample, and the sample was transferred to temperature cycle conditions for crystal maturation (except for the stage of holding at a constant temperature for crystal maturation, all other stages were carried out under magnetic stirring at a speed of approximately 1000 rpm).

The temperature cycle procedures were as follows:
first cycle: uniformly heating to 50°C in 0.3 hours, holding at a constant temperature of 50°C for 2.5 hours; cooling at a rate of 0.5°C/min to 5°C and holding at a constant temperature of 5°C for 2.5 hours;
second cycle: uniformly heating to 50°C in 0.4 hours, holding at a constant temperature of 50°C for 2.5 hours; cooling at a rate of 0.3°C/min to 5°C and holding at a constant temperature of 5°C for 2.5 hours; and
third cycle: uniformly heating to 50°C in 0.5 hours, holding at a constant temperature of 50°C for 2.5 hours; cooling at a rate of 0.1°C/min to 5°C and holding at a constant temperature of 5°C for 2.5 hours.

After a total of 3 cycles, solids were precipitated, and the resulting solid crystals were separated by centrifugation and, upon testing, were identified as the crystal form A.

### Example 4

Preparation of a crystal form A of 20(S)-protopanaxadiol:
The crude 20(S)-protopanaxadiol prepared in Example 1 was used as the raw material, approximately 19.8 mg of solids of the crude 20(S)-protopanaxadiol were weighed and placed in a 20 mL glass vial, and 0.8 mL of chloroform was added to dissolve the solids of the crude 20(S)-protopanaxadiol. The sample solution was filtered into a new 20 mL glass vial using a polytetrafluoroethylene filter membrane with a pore size of 0.45 µm, followed by magnetic stirring (at a speed of approximately 1000 rpm) to obtain a clear solution, 5 ml of n-hexane was added dropwise therein to obtain a gel-like sample, and the sample was transferred to temperature cycle conditions for crystal maturation (except for the stage of holding at a constant temperature for crystal maturation, all other stages were carried out under magnetic stirring at a speed of approximately 1000 rpm).

The temperature cycle procedures were as follows:
first cycle: uniformly heating to 50°C in 0.3 hours, holding at a constant temperature of 50°C for 1.5 hours; cooling at a rate of 0.5°C/min to 5°C and holding at a constant temperature of 5°C for 1.5 hours;
second cycle: uniformly heating to 50°C in 0.4 hours, holding at a constant temperature of 50°C for 1.5 hours; cooling at a rate of 0.3°C/min to 5°C and holding at a constant temperature of 5°C for 1.5 hours; and
third cycle: uniformly heating to 50°C in 0.5 hours, holding at a constant temperature of 50°C for 1.5 hours; cooling at a rate of 0.1°C/min to 5°C and holding at a constant temperature of 5°C for 1.5 hours.

After a total of 3 cycles, solids were precipitated, and the resulting solid crystals were separated by centrifugation and, upon testing, were identified as the crystal form A.

### Example 5

Preparation of a crystal form B of 20(S)-protopanaxadiol:
The crude 20(S)-protopanaxadiol prepared in Example 1 was used as the raw material, approximately 15 mg of solids of the crude 20(S)-protopanaxadiol were weighed and placed in a DSC aluminum dish. Under nitrogen purging conditions, the temperature was raised to 170°C at a heating rate of 10°C/min. The temperature was maintained constant for 3 minutes, and then cooled to 30°C at a cooling rate of 30°C/min. A total of 17 batches were set up in parallel. All solids were collected to obtain the crystal form B.

The X-ray powder diffraction pattern of the crystal form B of 20(S)-protopanaxadiol using Cu-Kα radiation is shown in figure 5.

The crystal form B of 20(S)-protopanaxadiol is characterized by the peak positions in pattern (Bragg angle 2θ, expressed in degrees), relative intensity (%), and interplanar spacing d, with the data shown in table 3.

When performing differential scanning calorimetry, the crystal form B began to melt when heated to around 219°C. Its DSC thermogram is shown in figure 6.

### Comparative Example 1

The crude 20(S)-protopanaxadiol prepared in Example 1 was used as the raw material, 19.7 mg of the crude 20(S)-protopanaxadiol was weighed and placed in a 2 mL glass vial, and 0.3 mL of tetrahydrofuran/n-heptane (a volume ratio of 1:1) was added to obtain a suspension. The suspension was magnetically stirred (with a speed of approximately 1000 rpm) at 50°C for about two hours, sealed, and cooled at a rate of 0.1°C/min from 50°C to 5°C. Then, it was allowed to stand at a constant temperature of 5°C for about a day to obtain solid crystals. After detection and characterization, the solid crystals prepared in this Comparative Example are in crystal form D.

### Comparative Example 2

The crude 20(S)-protopanaxadiol prepared in Example 1 was used as the raw material, 20.1 mg of solids of the crude 20(S)-protopanaxadiol were weighed and placed in a 3 mL glass vial, and 1.0 mL of acetonitrile was added to obtain a suspension. The suspension was magnetically stirred (with a speed of approximately 1000 rpm) at 50°C for about two hours, then sealed, and cooled at a rate of 0.1°C/min from 50°C to 5°C. Then, it was allowed to stand at a constant temperature of 5°C for about a day to obtain solid crystals. After detection and characterization, the solid crystals prepared in this Comparative Example are in crystal form D.

### Comparative Examples 3-6

The crude 20(S)-protopanaxadiol prepared in Example 1 was used as the raw material, approximately 20 mg of solids of the crude 20(S)-protopanaxadiol were weighed and placed in a 3 mL glass vial, and a corresponding volume of a solvent was added. The 3 mL glass vial was sealed with a sealing film. Four small holes were punched in the sealing film and left to evaporate at room temperature until solids were precipitated.

The detailed experimental conditions involved in the above Comparative Examples are shown in table 4.

**Table 4**

| Comparative Examples | Solid mass (mg) | Solvent | |
|---|---|---|---|
| | | Type (volume ratio) | Volume (mL) |
| 3 | 20.0 | Ethyl acetate | 1.0 |
| 4 | 19.8 | Acetone | 1.0 |
| 5 | 20.2 | Tetrahydrofuran | 1.0 |
| 6 | 19.7 | Acetonitrile | 1.0 |

After detection and characterization, the solid crystals prepared in Comparative Examples 3-6 are all in crystal form D.

### Comparative Example 7

The crude 20(S)-protopanaxadiol prepared in Example 1 was used as the raw material, approximately 20 mg of solids of the crude 20(S)-protopanaxadiol were weighed and placed in a 20 mL glass vial, and 0.6 mL of methyl isobutyl ketone was added to dissolve the solids of the crude 20(S)-protopanaxadiol. Subsequently, a clear solution was obtained by magnetic stirring (at a speed of approximately 1000 rpm). The solvent was allowed to slowly evaporate naturally, to precipitate solids, and the resulting solids were separated by centrifugation. After detection and characterization, the solid crystals prepared in this comparative example are in crystal form D.

### Test Example 1: Hygroscopicity test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the hygroscopicity of each test sample was measured, respectively.

The dynamic vapor sorption-desorption (DVS) curves of each sample were tested and plotted, respectively, and the experimental results are shown in table 5, wherein, the DVS profile of the crystal form A in Example 2 is shown in figure 7, the DVS profile of the crystal form B in Example 5 is shown in figure 8, and the DVS profile of the crude 20(S)-protopanaxadiol in Example 1 is shown in figure 9.

**Table 5**

| | Crystal form A | Crystal form B | Crystal form D |
|---|---|---|---|
| Hygroscopic weight gain at 25°C/80% RH (%) | 0.5763 | 0.2227 | 0.3863 |
| Crystal form change after DVS test | Unchanged | Unchanged | Unchanged |

According to the definition of drug hygroscopicity in the Chinese Pharmacopoeia 2020 edition, the crystal form A, crystal form B and crude 20(S)-protopanaxadiol described in the present invention all have slight hygroscopicity and can still maintain stable properties under high humidity conditions. During production, storage, and use, they can maintain stability without specific humidity control conditions, which can well meet the requirements of drug production and use.

Regarding the description of hygroscopicity characteristics and the definition of hygroscopic weight gain (Guidelines for Hygroscopicity Test of Drugs, Chinese Pharmacopoeia 2020 Edition):
Deliquescent: Absorbing sufficient moisture to form a liquid
Highly hygroscopic: Hygroscopic weight gain of not less than 15%
Hygroscopic: Hygroscopic weight gain of less than 15% but not less than 2%
Slightly hygroscopic: Hygroscopic weight gain of less than 2% but not less than 0.2%
Non-hygroscopic or almost non-hygroscopic: Hygroscopic weight gain of less than 0.2%.

### Test Example 2: Solubility Test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the solubility of each test sample was measured, respectively.

50 mg of each test sample was weighed, and a suspension was prepared using 5.0 mL of purified water, pH 1 buffer, SGF (simulated gastric fluid), FaSSIF (fasted state simulated intestinal fluid), and FeSSIF (fed state simulated intestinal fluid), respectively. After equilibration at 37°C for 1 hour, 2 hours, 4 hours, and 24 hours, filtration was performed to obtain clear solutions. The content of the sample in the saturated solution was measured by HPLC. The test results are shown in table 6.

The test results show that in purified water and pH 1 buffer medium, the 24-hour solubility of the crystal form A, crystal form B, and crude 20(S)-protopanaxadiol was relatively low. The 24-hour solubility of the crystal form A is 0.13 to 0.15 µg/mL, and the solubility of the crystal form B and crystal form D is lower than LOQ. In SGF, there is no significant difference in solubility among the crystal form A, crystal form B, and crude 20(S)-protopanaxadiol (crystal form D). In FaSSIF, the solubility of the crystal form A and crude 20(S)-protopanaxadiol (crystal form D) is higher than that of the crystal form B.

**Table 6**

| Medium | Time point | Crystal form A | Crystal form B | Crystal form D |
|---|---|---|---|---|
| Purified water (mg/mL) | 1 hour | <LOQ1 | <LOQ2 | <LOQ1 |
| | 2 hours | <LOQ1 | <LOQ2 | 0.00013 |
| | 4 hours | <LOQ1 | <LOQ2 | <LOQ1 |
| | 24 hours | 0.00013 | <LOQ2 | <LOQ1 |
| pH 1 buffer (mg/mL) | 1 hour | <LOQ1 | <LOQ2 | <LOQ1 |
| | 2 hours | <LOQ1 | <LOQ2 | <LOQ1 |
| | 4 hours | 0.00011 | <LOQ2 | <LOQ1 |
| | 24 hours | 0.00015 | <LOQ2 | <LOQ1 |
| SGF (mg/mL) | 1 hour | 0.020 | 0.018 | 0.021 |
| | 2 hours | 0.021 | 0.019 | 0.017 |
| | 4 hours | 0.017 | 0.018 | 0.016 |
| | 24 hours | 0.011 | 0.016 | 0.014 |
| FaSSIF (mg/mL) | 1 hour | 0.070 | 0.034 | 0.11 |
| | 2 hours | 0.073 | 0.039 | 0.072 |
| | 4 hours | 0.072 | 0.042 | 0.082 |
| | 24 hours | 0.12 | 0.042 | 0.10 |

| | | | | |
|---|---|---|---|---|
| LOQ1 is 0.1 µg/mL. LOQ2 is 0.3 µg/mL. | | | | |

### Test Example 3: Bulk density and tap density test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the bulk density and tap density of each test sample were measured, respectively.

Approximately 800 mg of each test sample was taken, and added into a 5 mL graduated cylinder, respectively, and the volume at this point was recorded. The graduated cylinder was lightly tapped against the tabletop 200 times, and the volume at this point was recorded. Three parallel tests were performed. The test results are shown in table 7.

**Table 7**

| Crystal form | Test No. | Bulk density (g/cm³) | Average bulk density (g/cm³) | Tap density (g/cm³) | Average tap density (g/cm³) |
|---|---|---|---|---|---|
| Crystal form A | 1 | 0.44 | 0.43 | 0.48 | 0.52 |
| | 2 | 0.46 | | 0.56 | |
| | 3 | 0.40 | | 0.51 | |
| Crystal form B | 1 | 0.22 | 0.21 | 0.25 | 0.25 |
| | 2 | 0.21 | | 0.24 | |
| | 3 | 0.21 | | 0.26 | |
| Crystal form D | 1 | 0.14 | 0.14 | 0.21 | 0.21 |
| | 2 | 0.14 | | 0.21 | |
| | 3 | 0.14 | | 0.20 | |

### Test Example 4: Repose angle test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the repose angle of each test sample was measured, respectively.

An appropriate amount of each test sample was taken. A funnel was fixed on an iron stand and positioned perpendicularly to the tabletop. The crystal form A and crystal form B samples were added into the funnel, respectively, allowing the sample to freely fall to form a cone. The height and diameter of the cone were measured to calculate the repose angle. Three parallel tests were performed. The test results are shown in table 8. The results indicate that the crystal form A has a smaller repose angle compared to the crystal form B and the crude 20(S)-protopanaxadiol, demonstrating better fluidity.

**Table 8**

| Crystal form | Test No. | Cone height (cm) | Cone diameter (cm) | Repose angle (°) | Average repose angle (°) |
|---|---|---|---|---|---|
| Crystal form A | 1 | 0.6 | 3.1 | 21.2 | 20.5 |
| | 2 | 0.5 | 2.9 | 19.0 | |
| | 3 | 0.6 | 3.1 | 21.4 | |
| Crystal form B | 1 | 0.7 | 3.0 | 25.0 | 24.2 |
| | 2 | 0.7 | 3.1 | 24.7 | |
| | 3 | 0.6 | 2.9 | 22.8 | |
| Crystal form D | 1 | 1.1 | 3.0 | 36.7 | 36.8 |
| | 2 | 1.2 | 3.1 | 38.2 | |
| | 3 | 1.0 | 2.8 | 35.5 | |

### Test Example 5: Contact angle test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the contact angle of each test sample was measured, respectively.

An appropriate amount of each test sample was taken, and its contact angle with water was measured. Two parallel tests were performed. The test results are shown in table 9.

**Table 9**

| Crystal form | Test 1 | Test 2 |
|---|---|---|
| Crystal form A | 70.7° - 74.0° | 73.7° - 78.3° |
| Crystal form B | 78.4° - 83.5° | 68.1° - 69.9° |
| Crystal form D | 124.8° - 125.2° | 129.8° - 129.8° |

### Test Example 6: Mechanical stability test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the mechanical stability of each test sample was measured, respectively.

An appropriate amount of each test sample was taken, placed respectively in an agate mortar, and manually ground for about 3 minutes, then, the crystal form was measured. The test results showed that the crystal form A, crystal form B, and crude 20(S)-protopanaxadiol were all transformed into amorphous form after grinding.

An appropriate amount of each test sample was taken, and added respectively into a tablet press. The crystal form was measured after tableting under a pressure of 200 MPa. The test results showed that the crystal form A, crystal form B, and crude 20(S)-protopanaxadiol all did not undergo crystal form transformation after tableting, but the crystallinity degree of both crystal form B and crude 20(S)-protopanaxadiol decreased, indicating that the crystal form A is superior to the crystal form B and crude 20(S)-protopanaxadiol.

### Test Example 7: Thermodynamic stability test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the thermodynamic stability of each test sample was measured, respectively.

An appropriate amount of the crystal form A was taken into a 2 mL glass vial. 0.5 mL of a corresponding solvent was added, and a suspension was obtained after magnetic stirring at a corresponding temperature for about 5 hours. The suspension was filtered at room temperature or 50°C, respectively, into a new 2 mL glass vial using a polytetrafluoroethylene filter membrane with a pore size of 0.45 µm. Approximately 5 mg of each of the crystal form A, crystal form B, and crude 20(S)-protopanaxadiol was weighed, respectively, and added into the filtered filtrate. Magnetic stirring was performed at a corresponding temperature. The test results showed that crystal form A was obtained after stirring the mixture of crystal form A, crystal form B, and crude 20(S)-protopanaxadiol at room temperature and 50°C. The test results are shown in table 10. The test results indicate that crystal form A is more stable than crystal form B and crude 20(S)-protopanaxadiol within the range of room temperature to 50°C.

**Table 10**

| Initial crystal form | Solvent (v/v) | Temperature | Time | Results |
|---|---|---|---|---|
| Mixture of crystal form A, crystal form B, and crystal form D | 2-butanone/n-heptane (1:4) | Room temperature | 4 days | Crystal form A |
| | 2-butanone/n-heptane (1:4) | 50 °C | 4 days | Crystal form A |
| | Ethyl acetate | Room temperature | 3 days | Crystal form A |
| | Ethyl acetate | 50 °C | 1 day | Crystal form A |

### Test Example 8: Solid-state stability test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and the solid-state stability of each test sample was measured, respectively.

An appropriate amount of each test sample was weighed, and subjected to stability tests under the following conditions: sealed storage at 80°C for 1 day, and open storage at 25°C/60% RH and 40°C/75% RH for 1 month, respectively. Solid samples separated under different conditions were subjected to XRPD to measure crystal form to evaluate physical stability, and HPLC to measure chemical purity to evaluate chemical stability. The evaluation results are summarized in table 11. The XRPD results showed that the crystal form A, crystal form B, and crude 20(S)-protopanaxadiol all did not undergo crystal form transformation under three conditions. The HPLC results showed that the purity of crude 20(S)-protopanaxadiol slightly decreased after sealed storage at 80°C for 1 day, and the chemical purity slightly decreased after open storage at 25°C/60% RH and 40°C/75% RH for 1 month; both crystal form A and crystal form B did not show a significant decrease in chemical purity under three conditions, indicating that crystal form A and crystal form B have better solid-state stability.

**Table 11**

| Crystal form (Sample No.) | Conditions | Purity (area%) | Crystal form change |
|---|---|---|---|
| Crystal form A | Initial | 97.21 | - |
| | 80°C/sealed/1 day | 97.20 | No |
| | 25°C/60% RH/open/1 month | 97.20 | No |
| | 40°C/75% RH/open/1 month | 97.18 | No |
| Crystal form B | Initial | 97.20 | - |
| | 80°C/sealed/1 day | 97.18 | No |
| | 25°C/60% RH/open/1 month | 97.17 | No |
| | 40°C/75% RH/open/1 month | 97.15 | No |
| Crystal form D | Initial | 96.01 | - |
| | 80°C/sealed/1 day | 94.04 | No |
| | 25°C/60% RH/open/1 month | 93.75 | No |
| | 40°C/75% RH/open/1 month | 93.00 | No |

### Test Example 9: Polarized light microscopy (PLM) test

In the present Test Example, the crude 20(S)-protopanaxadiol (crystal form D) prepared in Example 1, the crystal form A of 20(S)-protopanaxadiol prepared in Example 2, and the crystal form B of 20(S)-protopanaxadiol prepared in Example 5 were used as test samples, and PLM observation was performed on each test sample, respectively.

The PLM image of the crystal form A described in Example 2 is shown in figure 10. The PLM image of the crystal form B described in Example 5 is shown in figure 11. The PLM image of the crude 20(S)-protopanaxadiol described in Example 1 is shown in figure 12.

According to figures 10, 11, and 12, it can be observed under polarized light microscopy that crystal form A and crystal form B are mainly composed of irregular granular crystals, while crystal form D is mainly composed of irregular granular and needle-like/long flake-like crystals.

Although the present application has been described in detail with general explanations and specific embodiments in the preceding text, some modifications or improvements can be made based on the present application, which is obvious to a person skilled in the art. Therefore, all modifications or improvements made without departing from the spirit of the present application are within the scope of protection claimed by the present application.

### Industrial applicability

The present application provides crystal form A and crystal form B of 20(S)-protopanaxadiol, a preparation method therefor and the use thereof. The crystal form A and crystal form B provided in the present application are different from the existing known crystal forms of 20(S)-protopanaxadiol, each have a distinct profile, can be perfectly reproduced, have good bulk density, fluidity and stability, and good characteristics in terms of fighting depression, anxiety, cognitive disorder, tumor or cardiovascular diseases, and the like, and have good economic value and application prospects in the drug development of 20(S)-protopanaxadiol.

## Claims

1. A crystal form A of 20(S)-protopanaxadiol, wherein the X-ray powder diffraction pattern of the crystal form A, represented by 2θ ± 0.2° diffraction angles, exhibits characteristic diffraction peaks at least at 6.5°, 4.2° and 15.7°;
preferably, the X-ray powder diffraction pattern of the crystal form A, represented by 2θ ± 0.2° diffraction angles, further exhibits characteristic diffraction peaks at
(1) at least one of 17.1°, 18.6° and 13.1°; and/or
(2) at least one of 17.9°, 10.6° and 20.7°;
more preferably, the X-ray powder diffraction pattern of the crystal form A, represented by 2θ ± 0.2° diffraction angles, exhibits characteristic diffraction peaks at least at 6.5°, 4.2°, 15.7°, 17.1°, 18.6°, 13.1°, 17.9°, 10.6° and 20.7°.

2. The crystal form A of 20(S)-protopanaxadiol according to claim 1, wherein the melting point of the crystal form A is 219 to 221°C.

3. A preparation method for the crystal form A of 20(S)-protopanaxadiol according to claim 1 or 2, **characterized by** dissolving crude 20(S)-protopanaxadiol in a good solvent to obtain a 20(S)-protopanaxadiol solution, adding an antisolvent dropwise to the 20(S)-protopanaxadiol solution until a gel-like substance is formed, and subjecting the gel-like substance to at least one temperature cycle;
wherein the temperature cycle comprises: heating the gel-like substance to 45°C to 55°C, holding at a constant temperature for 1 to 3 hours, cooling at a rate of 0.05°C/min to 1°C/min to 4°C to 6°C, and holding at a constant temperature for crystal maturation for 1 to 3 hours;
preferably, the number of the temperature cycles is 2 to 4 times.

4. The preparation method according to claim 3, **characterized by** using a filter membrane with a pore size of 0.4 to 0.5 µm to filter the 20(S)-protopanaxadiol solution before adding the antisolvent dropwise; and/or, the good solvent is at least one selected from methyl isobutyl ketone, anisole and chloroform; the antisolvent is at least one selected from n-heptane, n-hexane and n-pentane.

5. A crystal form B of 20(S)-protopanaxadiol, wherein the X-ray powder diffraction pattern of the crystal form B, represented by 2θ ± 0.2° diffraction angles, exhibits characteristic diffraction peaks at least at 7.5°, 14.4° and 15.4°;
preferably, the X-ray powder diffraction pattern of the crystal form B, represented by 2θ ± 0.2° diffraction angles, further exhibits characteristic diffraction peaks at
(1) at least one of 4.6°, 3.8° and 16.9°; and/or
(2) at least one of 18.1°, 6.7° and 13.1°;
more preferably, the X-ray powder diffraction pattern of the crystal form B, represented by 2θ ± 0.2° diffraction angles, exhibits characteristic diffraction peaks at least at 7.5°, 14.4°, 15.4°, 4.6°, 3.8°, 16.9°, 18.1°, 6.7° and 13.1°.

6. The crystal form B of 20(S)-protopanaxadiol according to claim 5, wherein the XRPD data of the crystal form B is basically as shown in table 3; and/or, the XRPD pattern of the crystal form B is basically as shown in figure 5.

7. The crystal form B of 20(S)-protopanaxadiol according to claim 5 or 6, wherein the melting point of the crystal form B is 218°C to 220°C.

8. A preparation method for the crystal form B of 20(S)-protopanaxadiol according to any one of claims 5 to 7, **characterized by** heating crude 20(S)-protopanaxadiol first to 160°C to 180°C, maintaining for 2 to 5 minutes; and then cooling to 25°C to 35°C to obtain the crystal form B;
preferably, the heating rate is 8°C/min to 15°C/min; and/or, the cooling rate is 25°C/min to 35°C/min.

9. A pharmaceutical composition, comprising a therapeutic effective amount of the crystal form A according to any one of claims 1 to 2 or the crystal form B according to any one of claims 5 to 7; preferably, the pharmaceutical composition further comprising a pharmaceutically acceptable excipient.

10. Use of the crystal form A according to any one of claims 1 to 2, or the crystal form B according to any one of claims 5 to 7, or the pharmaceutical composition according to claim 9 in any one of the following applications:
(1) for the manufacture of a medicament for the treatment of depression, anxiety, cognitive disorder, tumor, or cardiovascular diseases;
(2) for treating depression, anxiety, cognitive disorder, tumor, or cardiovascular diseases; and
(3) for the manufacture of a medicament for the prevention and/or treatment of Alzheimer's disease (AD) and amyotrophic lateral sclerosis (ALS).
